# EUROPEAN PATENT SPECIFICATION

(11) **EP 3 753 530 B1**
(45) Date of publication and mention of the grant of the patent: **06.03.2024**
(21) Application number: 19750826.0
(22) Date of filing: 29.01.2019
(51) Int. Cl.: A61F 2/02, A61B 17/84

(54) **IMPLANT CAPABLE OF RELEASING DRUG, AND MANUFACTURING AND USAGE METHODS THEREFOR**
IMPLANTAT MIT FÄHIGKEIT ZUR FREISETZUNG EINES ARZNEIMITTELS UND VERFAHREN ZUR HERSTELLUNG UND VERWENDUNG DAVON
IMPLANT CAPABLE DE LIBÉRER UN MÉDICAMENT, ET PROCÉDÉS DE FABRICATION ET D'UTILISATION ASSOCIÉS

(30) Priority: 12.02.2018 HK 18102217
(43) Date of publication of application: 23.12.2020
(73) Proprietor: KOLN HONG KONG LIMITED, Wan Chai (HK)
(72) Inventor: FANG, Christian Xinshuo, Shatin, NT Hong Kong (HK); YAU, Wing Fung Edmond, Shatin, NT Hong Kong (HK); MAK, Sze Yi, Shatin, NT Hong Kong (HK); YUNG, Ching Hang Bob, Shatin, NT Hong Kong (HK)
(74) Representative: Loo, Chi Ching
(86) International application number: PCT/IB2019/050697
(87) International publication number: WO 2019/155321

(56) References cited:
- WO-A1-2011/029208
- CN-A- 104 203 220
- CN-A- 105 744 983
- CN-A- 105 832 653
- CN-A- 107 563 056
- CN-U- 204 864 514
- DE-A1-102015 205 538
- US-A1- 2004 225 360
- US-A1- 2010 042 214
- US-A1- 2010 217 401
- US-A1- 2017 027 628

## Description

### Technical Field

The invention relates to the technical field of medical appliances, in particular to a drug-releasing implant, the manufacturing and using method of this implant.

### Background of the Invention

At present, when the bone fracture occurs in a person, external fixation and internal fixation can be used as treatment. However, when the fracture is seriously damaged and the broken end is extremely unstable, the external fixation such as plastering and bracing may cause the fracture site to shift again. At this time, only the internal fixation can be used as treatment. The current method of internal fixation would depend on the condition of the patient. Intramedullary metal bone screw can be used to treat fractures by implanting the metal bone screw into the bone marrow, use the bone screw to fix the fractured bone; or use metal bone plate (orthopedic splint) and bone screw as fixing objects for the treatment of fractures, and use perforations on the metal bone plate to fix the fractured bone by the bone screw. In this way, it is necessary to drill the bone at the fracture site, then use one or more metal bone plates to connect the bones after the drilling is completed, and fix the metal bone plates with screws. When the fractured bone is healed, the metal bone screw or metal bone plate and screw should be decided whether to remove according to the situation. As the surgery should be taken, the fracture site is often very painful and might be inflamed especially 24 to 48 hours after the surgery. The pain is not only caused by the wound, but also comprising the psychological factor that affects the patient's rehabilitation progress. Generally, only internal drug can be used for pain relief and auxiliary treatment. Nevertheless, internal drug cannot avoid systemic administration and cannot meet the urgent needs of the majority of patients. CN104203220A disclosed devices and methods for administering a therapeutic agent, comprising an implantable delivery device. Exemplary embodiments comprise a reservoir in fluid communication with a channeled member, and a solid matter therapeutic agent disposed within the reservoir. CN204864514U disclosed a uterine medicine sustained-release device having pocket and gelinite. US20100042214A1 disclosed an orthopaedic implant system includes an orthopaedic implant implantable at a selected location within a corporeal body and configured for delivering at least one therapeutic agent to the corporeal body, the implant defining a reservoir and a plurality of channels, the reservoir configured for receiving the at least one therapeutic agent, the plurality of channels configured for conveying the at least one therapeutic agent from the reservoir to a treatment site relative to the corporeal body, the implant being at least one of an internal fixation device and a porous device. CN105832653A disclosed a biological absorbable foamed magnesium adhesive-matrix type targeted drug slow-release carrier and a preparation method. The carrier comprises a barrel body and a barrel cover, wherein one end of the barrel body is opened and the barrel cover is matched with the opened part; the barrel body is in threaded connection with the barrel cover; the barrel body and the barrel cover are prepared of foamed magnesium materials; communication micropores with pore diameters of 100-500 microns are uniformly distributed in the foamed magnesium materials and are used for slowly releasing drugs. After being filled with drugs, the carrier is implanted into pathological bone tissues, so as to realize the fixed-point controlled release and biodegradable absorption of the drugs and facilitate the ingrowth of new bone tissues. The mechanical properties of the foamed magnesium are similar to the mechanical properties of the natural bones, so that the foamed magnesium can be used at the bearing parts of human body. According to the preparation method, the foamed magnesium is prepared from polystyrene microspheres and magnesium powder by adopting a powder metallurgy method, the foamed magnesium is processed into a barrel with the barrel cover by adopting a machining method so as to form a capsule-shaped drug carrier, and MgF2 chemical conversion coatings with thicknesses of 1-2 microns are generated on the inner and outer surfaces of the carrier; the method is simple, convenient, feasible and high in reliability. US20170027628A1 disclosed a drug delivery implant implanted into a bone. The drug delivery implant includes an implant fixture provided with an inlet formed at the upper end thereof and a drug supply cartridge coupled to the fixture, the drug supply cartridge includes a cap to close the inlet of the fixture and a cartridge main body provided under the cap, coupled with the cap and accommodated in the fixture to release a drug, and a cartridge hole to accommodate the cartridge main body and drug channels to guide the drug released from the inside of the cartridge main body to the outside of the fixture are formed in the fixture. The drug delivery implant may continuously administer the drug into bone tissues and mount the drug cartridge together with the cap in the fixture, thus facilitating mount of the drug cartridge in the fixture and replacement of the drug cartridge.

### Summary of the Invention

In view of the deficiency of above prior art, the invention provides a drug-releasing implant that is convenient for fixation and increases the therapeutic effectiveness, such as pain relief, anti-inflammatory, repair, cancer treatment, etc., and can perform local application of drug to the fracture site at the same time.

Another purpose of the invention is to provide a manufacturing method of the above drug-releasing implant, so that customize appropriate implant according to the patient's fracture site, make the implant better fit with the fractured bone, in order to achieve good surgical effect.

Not in accordance with the present invention, there is provided a method of using the above drug-releasing implant.

The invention achieves above purposes in this way:
According to a first aspect of the invention, there is provided a drug-releasing implant, the implant is used to implant into a surgical site, said implant is provided with a drug cavity for storing drug, said drug cavity is filled with liquid drug; said implant is further provided with micro pipeline, the micro pipeline passes through the drug cavity and a surface of the implant, the drug in the drug cavity is released to the surgical site through the micro pipeline, characterised in that the drug cavity is set to a vacuum state and the implant is immersed in a drug bath, the drug penetrates into the drug cavity through the micro pipeline under the action of negative pressure.

Wherein, said micro pipeline may be the porous structure set on the wall between the implant and the drug cavity.

Wherein, said micro pipeline may be the net structure set on the wall between the implant and the drug cavity.

Wherein, said implant may be provided with screw mounting hole for fixing the implant to the surgical site, the locking screw can pass through the screw mounting hole to be fixedly connected to the surgical site.

According to a second aspect of the invention, there is provided a manufacturing method for the drug-releasing implant, wherein comprise the following steps:
A1. Obtain bone outline data of the surgical site;
A2. Calculate and generate the structural data of the implant according to the bone outline data, calculate the capacity of the drug cavity from the required drug dose after the surgery, and design the structural data of drug cavity and micro pipeline based on the structural data of the implant;
A3. Manufacture the implant by 3D printing method according to the structural data of implant, drug cavity and micro pipeline.

Not in accordance with the present invention, there is provided a using method for the drug-releasing implant, wherein comprise the following steps:
B1. Sterilize the implant and locking screw before the surgery;
B2. Anesthetize and sterilize the surgical site, then dissect the surgical site;
B3. Install the implant to the surgical site and adjust the implant to match the bone curvature of the surgical site, and then fix the implant to the surgical site with locking screw;
B4. Suture the surgical site;
Add the following Step C to B2 or B3: Inject the drug into the drug cavity.

Wherein, for said Step C, the wall between said implant and drug cavity is provided with injection hole, said drug is injected from the injection hole to the drug cavity by injector, then seal the injection hole.

Wherein, said Step C is to immerse the implant in the drug bath, the drug penetrates into the drug cavity through the micro pipeline.

Wherein, the drug cavity is set to a vacuum state and the implant is immersed in the drug bath, the drug penetrates into the drug cavity through the micro pipeline under the action of negative pressure.

Advantageous effect of the invention: The implant can effectively fix the bone at the surgical site (fracture site), after the surgery is completed, the drug set in implant's drug cavity slowly releases to the fracture site through micro pipeline, achieve local application of drug and reduce oral medication, so that decrease the fracture patients' medical expenses and psychological stresses, improve the utility of the device.

### Brief Description of the Drawings

Below further describes the invention combining with the drawings:
Figure 1 is the top view of the invention;
Figure 2 is the left view of the invention;
Figure 3 is the sectional view of the invention;
Figure 4 is the drawing of one embodiment of micro pipeline;
Figure 5 is the drawing of another embodiment of micro pipeline.

In the figures, 1. implant; 2. drug cavity; 3. micro pipeline; 4. screw mounting hole.

### Detailed Embodiment of the Invention

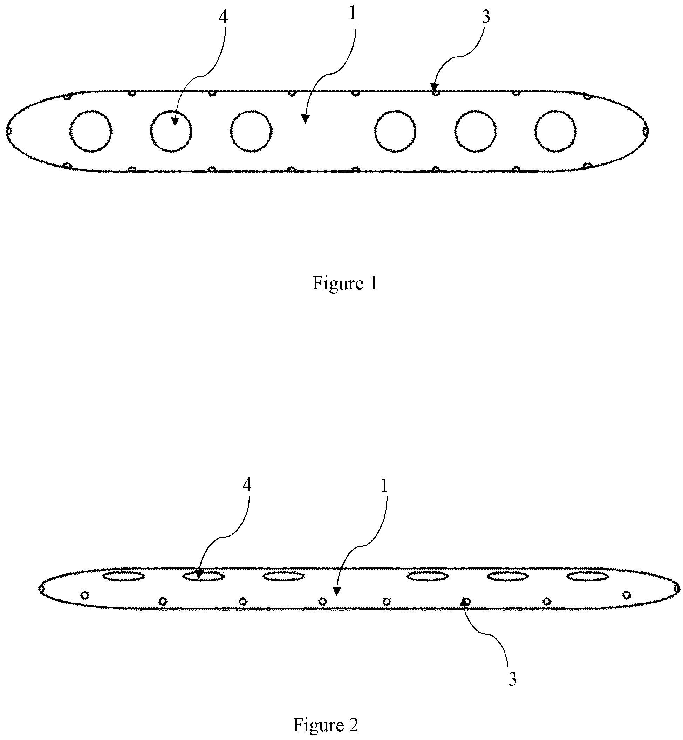

As shown in Figures 1-3, the drug-releasing implant comprises the implant 1 for fixing the surgical site, said implant 1 is provided with the drug cavity 2 for storing drug, said drug cavity 2 is filled with liquid drug; said implant 1 is further provided with micro pipeline 3, the micro pipeline 3 passes through the drug cavity 2 and the surface of the implant 1, the drug in the drug cavity 2 is slowly released to the surgical site through the micro pipeline 3. The implant 1 can effectively fix the bone at the surgical site fracture site, after the surgery is completed, the drug set in implant's 1 drug cavity 2 slowly releases to the fracture site through micro pipeline 3, achieve local application of drug and reduce oral medication, so that decrease the fracture patients' medical expenses and psychological stresses, improve the utility of the device.

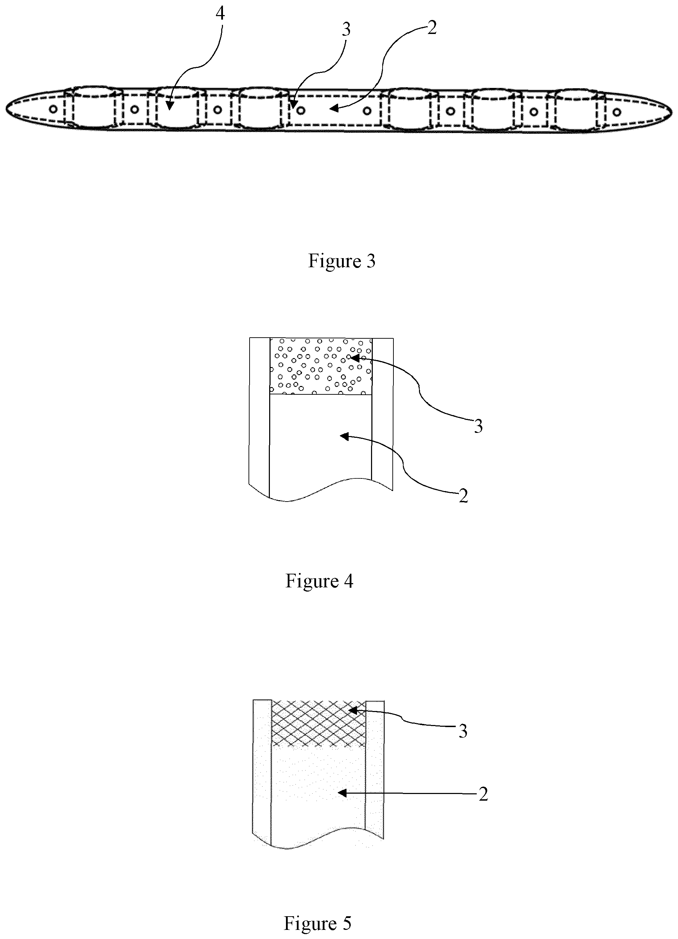

In order to release the drug in the drug cavity 2 conveniently, the micro pipeline 3 can be multiple structures, one is the porous structure set on the wall between the implant 1 and the drug cavity 2, refers to Figure 4. Another one is the net structure set on the wall between the implant 1 and the drug cavity 2, please refer to Figure 5.

Said implant 1 can be various devices used to fix the bone at the fracture site, comprising bone plate, bone screw (i.e. locking screws), etc. Wherein, the bone plate is used to connect and fix the fracture site, the bone screw is the device used to nail into the bone site to realize the connection between the bones or between the bone plate and the bone. The drug cavity 2 can be provided in both of the above, and the drug can be stored therein for slowly releasing to the fracture site after the surgery. In general, the overall size of bone plate is large, so the drug cavity 2 that can be provided is also large, and it is further provided with screw mounting hole at the same time, which is fixed to the bone by locking screw (i.e. bone screw). The size of the bone screw is small, only the small drug cavity 2 is provided, comprising bone marrow screw and bone screw.

The bone plate is taken as an example, detailed manufacturing steps and using method are described below.

As the outline of fractured bone in different sites and the site needing to be fixed are different, in order to make the bone plate better fit with the fracture site to achieve the most ideal surgery effect, the bone plate is manufactured by following steps: First of all, obtain the bone outline data of the surgical site through X-ray machine or magnetic resonance imaging equipment; then calculate and generate the structural data of the bone plate according to the bone outline data, and form the 3D model of bone plate through the computer, the wall thickness of bone plate can be 0.2-2mm; the required drug dose in every surgery is different in actual situation, and the site to where the drug is released is also different, thus the capacity of drug cavity 2 can be calculated based on above situation, and design the structural data of drug cavity 2 and micro pipeline 3 based on the structural data of the bone plate, the capacity of drug cavity 2 is generally 0.1-10ml, the volume ratio of drug cavity 2 to bone plate is 20% - 80%, the final bone plate 3D model can be formed by the computer; finally, manufacture the bone plate by 3D printing method according to the structural data of the bone plate, drug cavity 2 and micro pipeline 3. Said bone plate or locking screw should use the medical-grade metal to be the manufacture material, the metals are ductile and flexible to match the curvature of the bones at different sites, comprising medical-grade stainless steel, cobalt alloy or titanium alloy. The bone plate has two main surfaces, one is toward the surface of the bone, another one is toward the surface of the muscle; wherein the surface facing the bone is smooth and has a curve surface that fits the bone, the central axis of the bone plate is provided with several sets of locking screw holes, locking screws can be used to fix the bone plate on the fractured bone.

The using method of drug-releasing bone plate, before the surgery, sterilize the bone plate and locking screw in order to prevent the bacteria or virus infection; then anesthetize and sterilize the surgical site, and dissect the surgical site; install the bone plate to the surgical site and adjust the bone plate to match the bone curvature of the surgical site, and then fix the bone plate to the surgical site with locking screw; the wall between bone plate and drug cavity 2 is provided with injection hole, said drug is injected from the injection hole to the drug cavity 2 by injector, then seal the injection hole; suture the surgical site. Under the action of the human body fluids, microtubules, penetration and diffusion, the drug in the drug cavity 2 is required to release to the surgical site within 24 to 48 hours after the surgery, thereby reducing the pain of fracture patients, and can perform local application of drug to the fracture site to reduce oral medication at the same time. Regarding the use of injection method mentioned in above using method, that injecting the drug into the drug cavity 2, can also use the penetration method, such as penetrating the bone plate into the drug bath, the drug penetrates into the drug cavity 2 through the micro pipeline 3. For expediting the penetration speed of drug, the drug cavity 2 is set to a vacuum state and the bone plate is immersed in the drug bath, the drug penetrates into the drug cavity 2 through the micro pipeline 3 under the action of negative pressure.

Above contents are further described the invention combining with specific preferred embodiments, but cannot limit the embodiment of the invention to these only. For the common technicians who belong to the technical field of the invention, they can make some simple derivations or substitutions on the premise of not departing from the concept of the invention, it should be deemed as belonging to the protection scope of the invention.

## Claims

1. A drug-releasing implant, the implant (1) is used to implant into a surgical site, said implant (1) is provided with a drug cavity (2) for storing drug, said drug cavity (2) is filled with liquid drug; said implant (1) is further provided with micro pipeline (3), the micro pipeline (3) passes through the drug cavity (2) and a surface of the implant (1), the drug in the drug cavity (2) is released to the surgical site through the micro pipeline (3), **characterised in that** the drug cavity (2) is set to a vacuum state and the implant (1) is immersed in a drug bath, the drug penetrates into the drug cavity (2) through the micro pipeline (3) under the action of negative pressure.

2. The drug-releasing implant of Claim 1, wherein: said micro pipeline (3) is the porous structure set on the wall between the implant (1) and the drug cavity (2).

3. The drug-releasing implant of Claim 1, wherein: said micro pipeline (3) is the net structure set on the wall between the implant (1) and the drug cavity (2).

4. The drug-releasing implant of Claim 1, wherein: said implant (1) is provided with screw mounting hole (4) for fixing the implant (1) to the surgical site, the locking screw can pass through the screw mounting hole (4) to be fixedly connected to the surgical site.

5. A manufacturing method for the drug-releasing implant of any of Claims 1-3, wherein it comprises the following steps:
A1. Obtain bone outline data of the surgical site;
A2. Calculate and generate the structural data of the implant according to the bone outline data, calculate the capacity of the drug cavity (2) from the required drug dose after the surgery, and design the structural data of drug cavity (2) and micro pipeline (3) based on the structural data of the implant (1);
A3. Manufacture the implant (1) by 3D printing method according to the structural data of implant (1), drug cavity (2) and micro pipeline (3).

## Patentansprüche

1. Wirkstofffreisetzendes Implantat, wobei das Implantat (1) zum Implantieren in eine Eingriffsstelle verwendet wird, wobei das Implantat (1) mit einem Wirkstoffhohlraum (2) zum Aufbewahren eines Wirkstoffs versehen ist, wobei der Wirkstoffhohlraum (2) mit einem flüssigen Wirkstoff gefüllt ist; wobei das Implantat (1) weiterhin mit einer Mikropipeline (3) versehen ist, wobei die Mikropipeline (3) durch den Wirkstoffhohlraum (2) und eine Oberfläche des Implantats (1) verläuft, wobei der Wirkstoff in dem Wirkstoffhohlraum (2) durch die Mikropipeline (3) zu der Eingriffsstelle freigesetzt wird, **dadurch gekennzeichnet, dass** der Wirkstoffhohlraum (2) auf einen Vakuumzustand eingestellt ist und das Implantat (1) in ein Wirkstoffbad eingetaucht wird, wobei der Wirkstoff durch die Mikropipeline (3) unter der Einwirkung von Unterdruck in den Wirkstoffhohlraum (2) eindringt.

2. Wirkstofffreisetzendes Implantat nach Anspruch 1, wobei: die Mikropipeline (3) die poröse Struktur ist, die auf der Wand zwischen dem Implantat (1) und dem Wirkstoffhohlraum (2) gesetzt ist.

3. Wirkstofffreisetzendes Implantat nach Anspruch 1, wobei: die Mikropipeline (3) die Netzstruktur ist, die auf der Wand zwischen dem Implantat (1) und dem Wirkstoffhohlraum (2) gesetzt ist.

4. Wirkstofffreisetzendes Implantat nach Anspruch 1, wobei: das Implantat (1) mit einem Schraubenmontageloch (4) zum Befestigen des Implantats (1) an der Eingriffsstelle versehen ist, wobei die Feststellschraube durch das Schraubenmontageloch (4) hindurch gehen kann, um fest mit der Eingriffsstelle verbunden zu werden.

5. Herstellungsverfahren für das wirkstofffreisetzende Implantat nach einem der Ansprüche 1-3, wobei es die folgenden Schritte umfasst:
A1. Beziehen von Knochenkonturdaten der Eingriffsstelle;
A2. Berechnen und Erzeugen der Strukturdaten des Implantats gemäß den Knochenkonturdaten, Berechnen des Fassungsvermögens des Wirkstoffhohlraums (2) aus der erforderlichen Wirkstoffdose nach dem chirurgischen Eingriff und Entwerfen der Strukturdaten des Wirkstoffhohlraums (2) und der Mikropipeline (3) auf der Basis der Strukturdaten des Implantats (1);
A3. Herstellen des Implantats (1) durch ein 3D-Druckverfahren gemäß den Strukturdaten des Implantats (1), des Wirkstoffhohlraums (2) und der Mikropipeline (3).

## Revendications

1. Un implant libérant un médicament, l'implant (1) est utilisé pour être implanté dans un site chirurgical, ledit implant (1) est pourvu d'une cavité pour médicament (2) pour stocker le médicament, ladite cavité pour médicament (2) est remplie de médicament liquide ; ledit implant (1) est en outre pourvu d'un micro-pipeline (3), le micro-pipeline (3) traverse la cavité pour médicament (2) et une surface de l'implant (1), le médicament dans la cavité de médicament (2) est libéré jusqu'au site chirurgical à travers le micro-pipeline (3), **caractérisé en ce que** la cavité pour médicament (2) est maintenue à un état de vide et l'implant (1) est immergé dans un bain de médicament, le médicament pénètre dans la cavité pour médicament (2) à travers le micro-pipeline (3) sous l'action d'une pression négative.

2. L'implant libérant un médicament selon la revendication 1, dans lequel : ledit micro-pipeline (3) est la structure poreuse fixée sur la paroi entre l'implant (1) et la cavité pour médicament (2).

3. L'implant libérant un médicament selon la revendication 1, dans lequel : ledit micro-pipeline (3) est la structure en filet fixée sur la paroi entre l'implant (1) et la cavité pour médicament (2).

4. L'implant libérant un médicament selon la revendication 1, dans lequel : ledit implant (1) est pourvu d'un trou de montage de vis (4) pour fixer l'implant (1) au site chirurgical, la vis de verrouillage peut passer à travers le trou de montage de vis (4), être relié de manière fixe au site chirurgical.

5. Un procédé de fabrication pour l'implant libérant un médicament selon l'une quelconque des revendications 1 à 3, dans lequel il comprend les étapes suivantes :
Al. Obtenir des données sur le contour osseux du site chirurgical ;
A2. Calculer et générer les données structurelles de l'implant en fonction des données de contour osseux, calculer la capacité de la cavité pour médicament (2) à partir de la dose de médicament requise après la chirurgie et concevoir les données structurelles de la cavité pour médicament (2) et du micro-pipeline ( 3) sur la base des données structurelles de l'implant (1) ;
A3. Fabriquer l'implant (1) par méthode d'impression 3D selon les données structurelles de l'implant (1), de la cavité pour médicament (2) et du micro-pipeline (3).
